(19) Europäisches Patentamt / European Patent Office / Office européen des brevets

(11) **EP 3 310 899 B1**

(12) **EUROPEAN PATENT SPECIFICATION**

(45) Date of publication and mention
of the grant of the patent:
**25.05.2022  Bulletin 2022/21**

(21) Application number: **16815097.7**

(22) Date of filing: **17.06.2016**

(51) International Patent Classification (IPC):
**C12M 1/34** *(2006.01)*      **G01N 21/64** *(2006.01)*
**G06T 7/00** *(2017.01)*      **B01L 3/00** *(2006.01)*
**B01L 7/00** *(2006.01)*

(52) Cooperative Patent Classification (CPC):
**B01L 3/5027; B01L 3/502707; B01L 3/502715;
B01L 7/52; G06T 7/0012; G06T 7/0016;**
B01L 2200/04; B01L 2200/0684; B01L 2200/0689;
B01L 2300/0645; B01L 2300/0816;
B01L 2300/0867; B01L 2300/0887; B01L 2300/161;
B01L 2400/049;                              (Cont.)

(86) International application number:
**PCT/US2016/038157**

(87) International publication number:
**WO 2016/209735 (29.12.2016 Gazette 2016/52)**

(54) **CAMERA IMAGING SYSTEM FOR A FLUID SAMPLE ASSAY AND METHOD OF USING SAME**

KAMERAABBILDUNGSSYSTEM FÜR FLÜSSIGPROBENTEST UND VERFAHREN ZUR NUTZUNG DESSELBEN

SYSTÈME D'IMAGERIE À CAMÉRA POUR DOSAGE D'ÉCHANTILLON DE FLUIDE ET PROCÉDÉ D'UTILISATION ASSOCIÉ

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **22.06.2015  US 201562182992 P
01.07.2015  US 201562187471 P**

(43) Date of publication of application:
**25.04.2018  Bulletin 2018/17**

(73) Proprietor: **FluxErgy, LLC
Irvine CA 92618 (US)**

(72) Inventors:
• **PATEL, Tej
  Carlsbad, CA 92011 (US)**
• **REVILLA, Ryan
  Costa Mesa, CA 92626 (US)**
• **HELTSLEY, Roy
  Irvine, CA 92618 (US)**

(74) Representative: **Mitchell, Simon James
Murgitroyd & Company London
Euston House
24 Eversholt Street
London NW1 1AD (GB)**

(56) References cited:
**WO-A1-2007/035172        WO-A1-2014/055963
WO-A1-2014/055963        WO-A1-2015/032835
US-A1- 2008 253 633        US-A1- 2012 140 055
US-A1- 2012 309 010        US-A1- 2014 307 931
US-B1- 7 867 754          US-B2- 8 158 926**

EP 3 310 899 B1

(52) Cooperative Patent Classification (CPC): (Cont.)
G01N 21/6456; G06T 2207/10016;
G06T 2207/10024; G06T 2207/10064;
G06T 2207/20021; G06T 2207/30072

## Description

FIELD OF THE DISCLOSURE

The present disclosure relates generally to apparatuses and methods for performing an assay, and more specifically to apparatuses and methods for causing a point-of-care polymerase chain reaction and analyzing the polymerase chain reaction at the point-of-care.

BACKGROUND OF THE DISCLOSURE

Point-of-care (POC) *in vitro* diagnostics tests (IVDT) have traditionally had two major categories, nucleic acid amplification tests (NAAT) or immunoassay-based tests. The former directly detects the pathogen's DNA or RNA, while the latter detects antibodies or antigens generated by the immune system response to the pathogen.

Current POC diagnostic immunoassays lack the high sensitivity and specificity of nucleic acid amplification methods. This becomes more pronounced during the initial stages of infection, often within 168 hours. Taking the case of Dengue virus in whole blood, immunoglobulin M (IgM) and immunoglobulin G (IgG) remain undetectable in the majority of patients until 5 and 10 days post-infection, respectively, whereas nucleic acid can be found as early as 0 to 7 days. Moreover, many immunoassay tests are unable to detect infectious agents until 3 months after the initial onset of the infection. This delay is due to the time it takes for the body's immune system to respond to an infection.

POC diagnostic assays developed utilizing NAATs have very high sensitivities and specificities, matching those of currently accepted laboratory tests. The primary mechanism of NAAT based systems is to directly detect an infectious agent's nucleic acid, lending to the test's ability to detect diseases within the first few days of the onset of infection. In addition, by careful primer design, NAATs also have the ability to have very high specificity and sensitivity compared to immunoassay based testing. The largest drawback of NAATs compared to immunoassay-based tests is the complicated equipment and/or processes required to prepare a sample for testing.

WO/2007/035172 of Attogenix Biosystems Pte Ltd describes a method and apparatus for analysing a sample fluid. The apparatus may include a microfluidic device having at least one reaction chamber having a cover transparent to light. Each of the at least one reaction chambers is for receiving therein the sample fluid for analysis such that the sample fluid at least substantially fills the reaction chamber, thereby avoiding mist or condensation forming at the inner surface of the cover when the fluid is heated. Alternatively, the apparatus may be furnished with heating and/or imaging capabilities, including an image processing system for observing artefacts, identifying reaction areas from non-reaction areas, recognising reaction chamber boundaries, and/or detecting regions containing no artefacts and/or bubbles. This is carried out through an analysis of intensity variation in the collected image data and comparison with an established library of reaction chamber data.

WO/2015/032835 of Maersk Olie OG Gas A/S describes a method and a system for automatic detection of fluid inclusions in crystalline materials, such as rocks. The method comprises receiving at least one digital image of a crystalline material, determining global image intensity properties of each pixel of the received digital image and applying one or more global image filters on the determined global image intensity properties to provide a first filtered image. A set of filters are successively applied by segmenting the first or a further filtered image into a plurality of sections, applying a filter from the set of filters on one or more determined intensity properties for each of the plurality of sections to provide a further filtered image. A resulting filtered image is provided and based on the resulting filtered image, fluid inclusions in the at least one received image are identified.

WO2014055963 of California Institute of Technology discloses a device for analysing a polymerase chain reaction in a fluid sample.

SUMMARY OF THE DISCLOSURE

A first aspect of the invention provides a device for analyzing a polymerase chain reaction in a fluid sample, the device comprising: a current source (30) configured to cause the polymerase chain reaction by heating the fluid sample within a target zone (166); a camera imaging device (20) configured to record a plurality of images of the fluid sample in the target zone while the current source causes the polymerase chain reaction; and a controller (28) configured to, for at least one of the plurality of images, (i) virtually divide the target zone in the respective image into a plurality of bins (200) without the bins being separated by physical barriers, (ii) distinguish wanted objects in the plurality of bins from an unwanted object in the plurality of bins by comparing a size or shape of objects in the respective image to an average size or an average shape of blood cells, and (iii) determine whether the fluid sample tests positive or negative for a bacteria or virus based on the wanted objects by selecting at least two of the plurality of bins that overlap a cluster of wanted objects and calculating a mean fluorescence value of the at least two of the plurality of bins, wherein the wanted objects include cells, and the unwanted object includes an object that does not have at least one of the average size or the average shape of the cells, and wherein the controller (28) is configured to at least one of (a) exclude at least one of the plurality of bins from the mean fluorescence value calculation if the at least one of the plurality of bins overlaps the unwanted object, or (b) assign a weight to at least one of the plurality of bins used in the mean fluorescence value calculation based on the proximity of the at least one of the plurality of bins to the unwanted object.

In an embodiment, the unwanted object is an air bubble.

In an embodiment, the controller (28) is configured to assign weights to at least two of the plurality of bins based on a proximity of the unwanted object, the weights being greater than zero, and use the weights of the at least two of the plurality of bins in a mean fluorescence value calculation.

In an embodiment, the plurality of bins (200) are arranged in a grid with a plurality of rows and columns.

In an embodiment, the controller (28) is configured to additionally select the at least two of the plurality of bins based on a threshold value for brightness.

In an embodiment, the controller (28) is configured to additionally exclude at least one of the plurality of bins if the at least one of the plurality of bins does not meet a minimum threshold value for brightness.

In an embodiment, the controller (28) is configured to report an inconclusive test if the controller identifies an unwanted image in the plurality of images.

In an embodiment, the device includes a user interface (60), the controller (28) includes a plurality of preprogrammed analyses that can be performed on the fluid sample, and the user interface is configured to allow a user to select at least one analysis from the plurality of preprogrammed analyses.

In an embodiment, the plurality of images includes at least one of: (i) a plurality of still images of the polymerase chain reaction recorded by the camera imaging device (20) over a period of time; or (ii) a video image of the polymerase chain reaction recorded by the camera imaging device over the period of time.

In an embodiment, the device (10) further comprising a light source (22) that provides a fluorescent excitation light on the fluid sample.

In an embodiment, the device (10) is configured to receive a test card (100) including the fluid sample, and wherein the target zone is located within the test card.

A second aspect of the invention provides a method of using the device according to the invention, which includes: heating the fluid sample in the target zone (166) to cause the polymerase chain reaction; recording the plurality of images of the fluid sample in the target zone during the polymerase chain reaction while a light source (22) provides a fluorescent excitation light on the fluid sample; dividing the target zone into a plurality of bins in at least one of the plurality of images; calculating a fluorescence value using at least two of the plurality of bins for the respective image; and determining whether the fluid sample tests positive or negative for a bacteria or virus based on the calculated fluorescence value by selecting at least two of the plurality of bins that overlap a cluster of wanted objects and calculating a mean fluorescence value of the at least two of the plurality of bins, wherein the wanted objects include cells, and the unwanted object includes an object that does not have at least one of the average size or the average shape of blood cells, and wherein calculating the fluorescence value includes at least one of (i) weighting the at least two of the plurality of bins based on proximity to an unwanted object, or (ii) excluding at least one of the plurality of bins from the fluorescence value calculation if the at least one of the plurality of bins overlaps an unwanted object.

The invention is defined in the appended claims. Additional non-claimed subject matter is disclosed for reference.

BRIEF DESCRIPTION OF THE DRAWINGS

The present invention will now be explained in further detail by way of example only with reference to the accompanying figures, in which:

FIG. 1 is a top perspective view of an example embodiment of an assay device according to the present disclosure;

FIG. 2 is a top perspective view of an example embodiment of a test card according to the present disclosure;

FIG. 3 is a cross-sectional view of the test card of FIG. 2;

FIG. 4 is a top perspective view of the assay device of FIG. 1 with the test card of FIG. 2 inserted therein;

FIG. 5 is a cross-sectional view of the assay device and test card shown in FIG. 4;

FIGS. 6A to 6C illustrate an example embodiment of amplified images showing a PCR;

FIGS. 7A to 7E illustrate an example embodiment showing how a controller can detect bubbles and analyze a reaction even when bubbles are detected;

FIG. 8 illustrates an example embodiment of a control method that can be used to perform and analyze a reaction according to the present disclosure;

FIG. 9 illustrates an example embodiment of a controller that can perform the method of FIG. 8.

DETAILED DESCRIPTION

Before describing in detail the illustrative system and method of the present disclosure, it should be understood and appreciated herein that the present disclosure relates to a rapid, high sensitivity and high specificity, low complexity, diagnostic system 1 using nucleic acid amplification and capable of operating in low resource settings with minimal user training. The system described herein is configured, for example, to cause and analyze polymerase chain reactions (PCR), particularly in the early stages of infection, using a low-cost microfluidic platform employing PCR with a modified DNA polymerase.

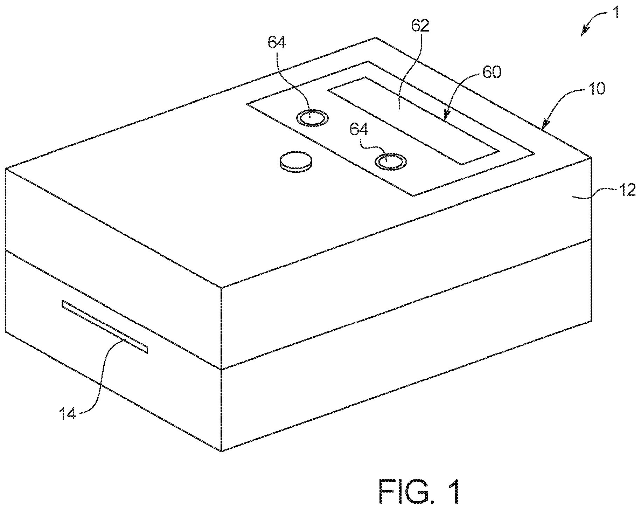

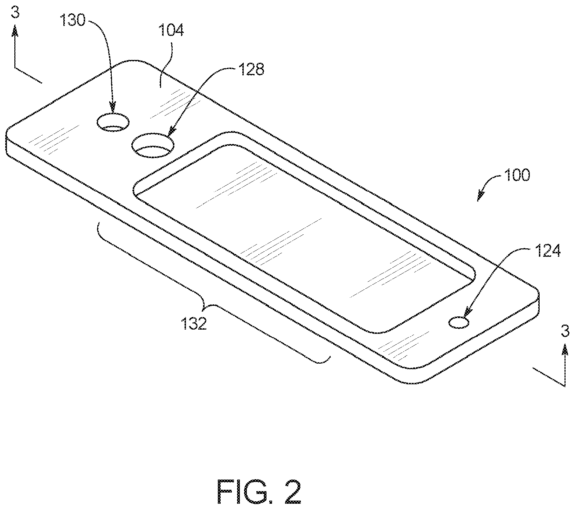

FIG. 1 illustrates an example embodiment of a point-of-care diagnostic system 1 according to the present disclosure. As illustrated, diagnostic system 1 includes an assay device 10 with a housing 12 having a slot 14 to receive a test card 100 (FIG. 2), which is an inexpensive, disposable test card that can be used with device 10 and then discarded. As explained in more detail below, a fluid sample can be injected into test card 100, and then test card 100 can be inserted into slot 14 so that device 10 can power test card 100 to run an assay within test card 100 without further action by the user. The resulting analysis can then be displayed to the user by user interface 60. Test card 100 can then be discarded and a new test card 100 can be inserted into slot 14 and used the same way to run a new assay. The test card may be configured to receive about 10 µL of whole blood, the equivalent to a drop of blood obtained from a finger stick. Alternatively, the fluid sample may be serum, urine, saliva, tears and/or the like.

Device 10 is described in more detail in U.S. Application No. 15/185,640 (published as US 2016/0369322 A1), entitled "Device for Analyzing a Fluid Sample and Use of Test Card with Same".

Test card 100 is described in more detail in U.S. Application No. 15/185,661 (published as US 2016/0369323 A1), entitled "Test Card for Assay and Method of Manufacturing Same".

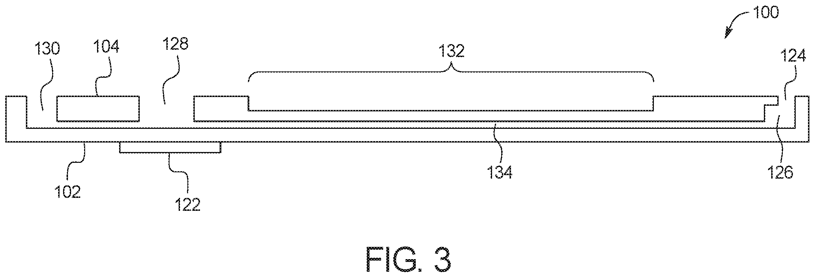

As illustrated in FIGS. 2 and 3, test card 100 includes an inlet port 124, a mixing chamber 126, a capture port 128, an outlet port 130, and a fluid microchannel 134. A liquid sample can be injected into inlet port 124 and mixed with one or more reagent in mixing chamber 126, and then test card 100 can be placed into slot 14 of assay device 10. Once test card 100 has been placed within device 10, the fluid sample can be pulled though fluid microchannel 134, so that the fluid sample can be analyzed through an analysis port 132 of test card 100. Test card 100 also includes electrical contacts 122 on a bottom surface 102 thereof, which enables electrodes adjacent to fluid microchannel 134 to be controlled to power test card 100, for example, to heat fluid within fluid microchannel 134, track fluid flow through fluid microchannel 134 and/or measure the concentration of a chemical species in the fluid sample.

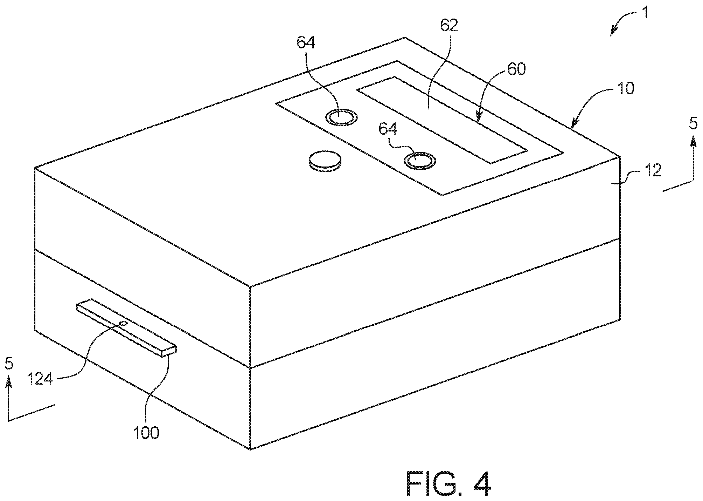

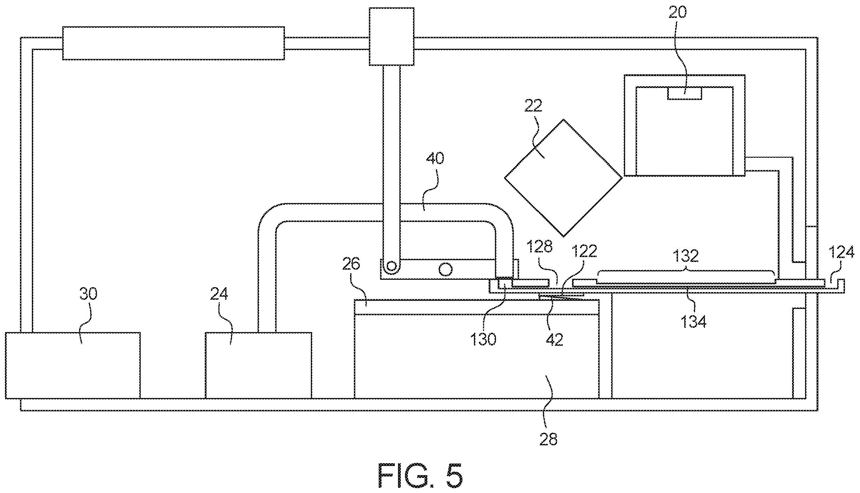

FIG. 4 illustrates a perspective view of device 10 after test card 100 has been placed into slot 14, and FIG. 5 illustrates a cross-sectional view thereof. As illustrated, placement of test card 100 into slot 14 aligns several of the elements of device 10 with several of the elements of test card 100. For example, placement of test card 100 into slot 14 aligns camera imaging device 20 and light source 22 of device 10 with analysis port 132 on an upper surface 104 of test card 100, pneumatic tube 40 of fluid actuation source 24 with outlet port 130 on the upper surface 104 of test card 100, and electrical contacts 42 of electrical contact device 26 with electrical contacts 122 on the bottom surface 102 of test card 100.

As explained in more detail in U.S. Application No. 15/185,640 (published as US 2016/0369322 A1), once pneumatic tube 40 is sealed against outlet port 130, a negative pneumatic force can be applied to outlet port 130 from fluid actuation source 24. When the negative pneumatic force is applied, the fluid sample injected into inlet port 124 is pulled through fluid microchannel 134 towards outlet port 130. The fluid sample however is not pulled into pneumatic tube 40 due to the presence of capture port 128 between inlet port 124 and outlet port 130. Capture port 128 allows fluid to build up before it can reach outlet port 130 and/or pneumatic tube 40, which keeps device 10 sterile and protects the integrity of diagnostic system 1.

Test card 100 is dimensioned so that electrical contacts 122 of test card 100 are placed into electrical contact with electrical contacts 42 of electrical contact device 26 when test card 100 is fully inserted into slot 14. With the electrical contacts 42 and 122 aligned, controller 28 can perform several functions. Before beginning an assay, controller 28 can ensure that the fluid sample has been properly pulled through microchannel 134 and into a target zone of microchannel 134 by measuring the capacitance of the fluid sample upstream and/or downstream of the target zone. Once it is determined that fluid is located within the target zone of microchannel 134, controller 28 can control power source 30 to apply a current to electrical contacts aligned with the target zone to heat the fluid sample located within target zone and cause a reaction such as a PCR to occur.

The alignment of camera imaging device 20 over analysis port 132 on the upper surface 104 of test card 100 allows controller 28 to analyze one or more reaction within fluid microchannel 134 while controlling the reaction. Camera imaging device 20 is configured to record a plurality of images of the fluid sample within the target zone so that the images can be analyzed in real time. The plurality of images can include, for example, a plurality of still images and/or a video image. Camera imaging device 20 is therefore configured to record the reaction of the fluid sample by taking a series of still images of the fluid sample within the target zone during a reaction or by taking a video of the fluid sample within the target zone during a reaction. As explained in more detail below, the plurality of images can be used, for example, to analyze the physical and/or chemical characteristics of cells within the fluid sample.

The target zone can be anywhere along fluid microchannel 134 or can be branched off of fluid microchannel 134. Examples of target zones are described in more detail in U.S. Application No. 15/185,661 (published as US 2016/0369323 A1), entitled "Test Card for Assay and Method of Manufacturing Same". In an aspect of the disclosure, the target zone can be located at a central portion of fluid microchannel 134 and fluid microchannel 134 can include, for example, a capacitance sensor upstream and/or downstream of the target zone to determine whether fluid has been pulled through fluid microchannel 134 and into the target zone. Microchannel 134 may include a plurality of target zones to perform a plurality of assays at the same or different times, for example, to improve the reliability of the assays. Controller 28 may be programmed to analyze a specific section of microchannel 134 as the target zone, or controller 28 can determine the target zone based on the plurality of images taken by camera imaging device 20.

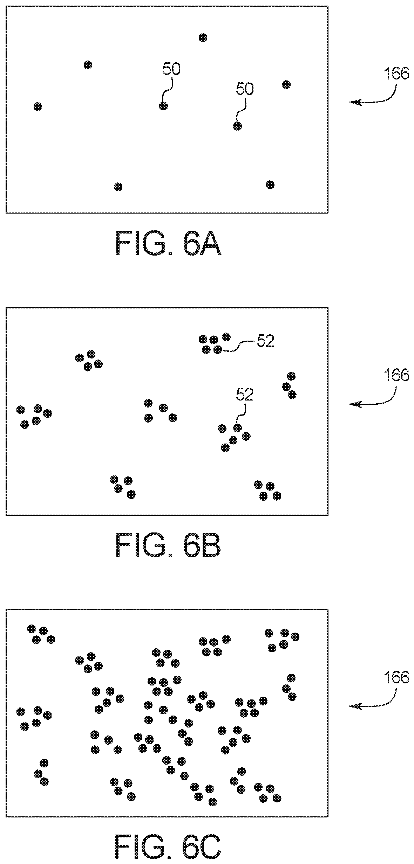

FIGS. 6A to 6C illustrate an example embodiment of amplified images showing a PCR. FIG. 6A illustrates a fluid sample that has been initially loaded into target zone 166, with a few nucleic acid molecules 50 separated by large distances. FIG. 6B shows the fluid sample during the PCR, when clear colonies 52 of nucleic acid molecules 50 can be viewed because the diffusion speed of the molecules is slower than the speed required for the molecules to travel the distance between colonies. FIG. 6C shows the end of the PCR, when the colonies 52 are no longer clearly visible.

From the number of colonies shown in FIG. 6B, controller 28 can determine whether the fluid sample tests positive or negative for a specific virus or bacteria based on a known titer value for the specific virus or bacteria. The titer value corresponds to the highest dilution factor for the specific virus or bacteria that yields a positive test result. By counting the colonies in FIG. 6B, and by comparing the count with the known titer value, controller 28 can determine a positive or negative test result for the fluid sample. The known titer value can be compared through a variety of methods. A statistical analysis may be utilized to say with certain probability that the result will fall under a certain titer value. This can be achieved by running a statistically significant amount of controlled samples under controlled dilutions and generate a standard curve based on the crossover threshold value of each (point at which the curve starts to increase).

Camera imaging device 20 may include a high sensitivity and dynamic range complementary metal-oxide semiconductor (CMOS) camera sensor which allows for general imaging of a reaction within target zone 166 of fluid microchannel 134 of test card 100. The CMOS camera sensor may enable camera imaging device 20 to image the entire analysis port 132 of test card 100. Although in the illustrated embodiment only a single target zone 166 within a single microchannel 134 is shown, camera imaging device 20 is configured to image a plurality of target zones and/or a plurality of microchannels should the user insert such a test card 100 into device 10. Examples of alternative test cards 100 are disclosed in U.S. Application No. 15/185,661 (published as US 2016/0369323 A1), entitled "Test Card for Assay and Method of Manufacturing Same".

In the illustrated embodiment, light source 22 is configured to project a fluorescent excitation light on target zone 166 of fluid microchannel 134 during a PCR, while camera imaging device 20 is recording still and/or video images of the PCR. The fluid sample may have been mixed with a fluorescence reagent in mixing port 126, before the fluid sample is pulled into target zone 166. By illuminating target zone 166 with a fluorescence excitation light, controller 28 can record fluorescence measurements from the images taken by camera imaging device 20. The fluorescence images can be analyzed to determine the incremental increases in fluorescence during each PCR cycle. This allows the determination of the PCR amplification curve. Additionally, the images can be used during melt analysis to determine the incremental decrease in fluorescence as temperature is increased. Incremental change in fluorescence can be determined by taking the difference in pixel intensity between successive images.

For general fluorescence measurements, for example, a statistical formulation of the PCR can be achieved by subdividing the target zone 166 and measuring florescence at various locations in the target zone 166. This yields highly-accurate and consistent crossover threshold values for the real-time PCR based on titer values. Once the PCR is complete, a melting curve analysis allows for error checking, ensuring that the correct amplicon has been amplified during the PCR, to reduce the likelihood of a false-positive test.

Camera imaging device 20 also enables controller 28 to record a variety of other measurements in addition to fluorescence measurements. For example, controller 28 can determine turbidity and object detection using the images taken by camera imaging device 20. The turbidity can be determined by using the device LEDs to apply incident light to a test card microchannel and/or target zone. The amount of scattered light can be measured by the camera allowing a numerical value of relative turbidity to be measured. The turbidity can be used to determine the outcome of various different reactions, such as measuring the reactiveness of proteins during an ELISA test (colorimetry measurement) or measurement of water quality. The object detection can be used, for example, to determine whether air bubbles formed in target zone 166 during the PCR and potentially destroyed the integrity of the PCR analysis. By imaging the entire analysis port 132 of test card 100, camera imaging device 20 is able to greatly improve accuracy by allowing for various advanced image-processing algorithms to be applied.

With respect to object detection, camera imaging device 20 can be used to detect both wanted and unwanted objects within microchannel 134. The wanted objects may be cells such as red and white blood cells in the fluid sample to be analyzed during the reaction. Controller 28 can count the wanted objects by matching the cell size and cell shape of objects in the images from camera imaging device 20 to the average size and shape of specific blood cells, allowing controller 28 to count, for example, the number of red and white blood cells in the fluid sample at target zone 166. Controller 28 can also distinguish the red and white blood cells from other unwanted objects that do not match the average size and shape of specific blood cells.

If controller 28 detects an unwanted object, controller 28 can determine that the PCR has failed or is indeterminate, and can instruct a user that an additional PCR should be run on the same or a different test card. If the PCR is run on the same test card, target zone 166 should be cleared of fluid, and then new fluid should be pulled from mixing chamber 126 into target zone 166.

In an embodiment, the unwanted image is an air bubble. In an aspect of the disclosure, controller 28 can detect air bubbles because air bubbles will expand in the target zone 166 while the PCR reaction occurs. Red and white blood cells, on the other hand, multiply but do not increase in size. Controller 28 can therefore determine the presence of an air bubble in target zone 166 by noting a change in size of an object across a plurality of subsequent images from the plurality of images. In another aspect of the disclosure, controller 28 can determine the presence of an air bubble by noting that an unwanted object does not match the average size and shape of blood cells. In another aspect of the disclosure, controller 28 can determine the presence of an air bubble due to there being no fluorescence inside of the bubble by looking for a change in the gradient of fluorescence.

In some cases, a bubble formed within microchannel 134 will remain small and will not affect any fluid flow or reaction occurring within microchannel 134. When heat is applied to target zone 166, however, the heat can cause the bubble to expand due to the large thermal expansion coefficient of the bubble.

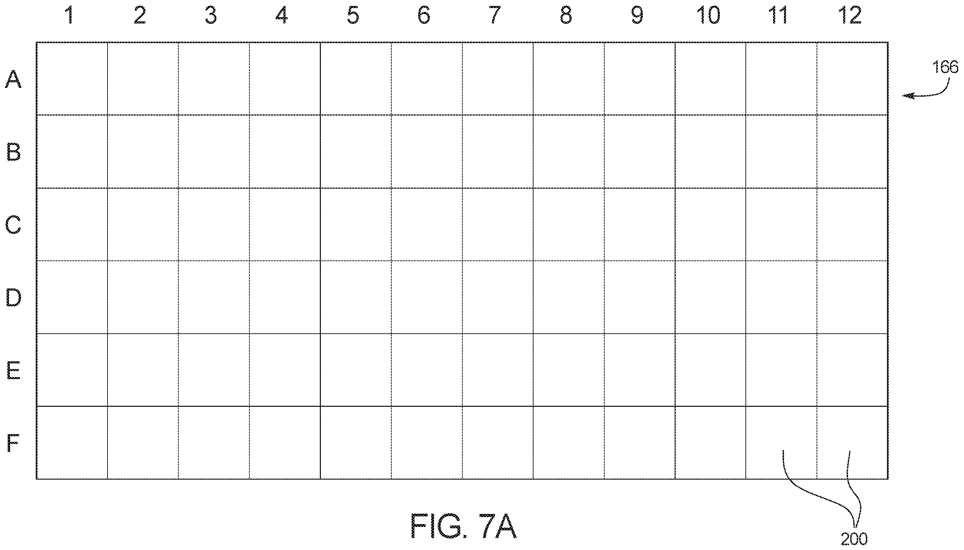

FIGS. 7A to 7E illustrate an example embodiment showing how controller 28 can detect unwanted objects such as bubbles and analyze a reaction even when bubbles are present. In some cases, unwanted objects may cause the reaction to fail completely and need to be rerun. A failure might occur if there are too many bubbles to compensate for. In FIGS. 7A to 7E, however, controller 28 can proceed with the reaction and analysis even with an air bubble present.

FIG. 7A illustrates an example embodiment of target zone 166 before any fluid has entered the target zone. As illustrated, controller 28 has divided target zone 166 into a plurality of bins 200. In the illustrated embodiment, controller 28 has divided target zone 166 into six rows (labeled A to F for ease of reference) and twelve columns (labeled 1 to 12 for ease of reference) to create seventy-two bins 200, but those of ordinary skill in the art will recognize that any number of bins 200 can be created within a target zone 166. It should be understood that the bins 200 are created virtually by controller 28, and there are no physical barriers separating the bins 200 within target zone 166.

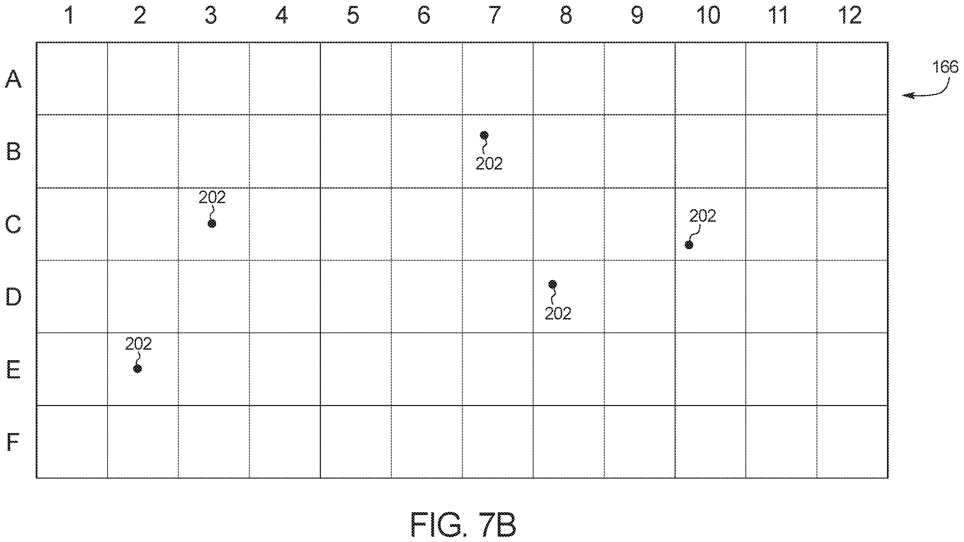

FIG. 7B illustrates target zone 166 after receiving a fluid sample but before a reaction has occurred. As illustrated, there are a minimal number of nucleic acid molecules 202 in the fluid sample before the reaction. In the illustrated embodiment, bins B7, C3, C10, D8 and E2 contain nucleic acid molecules 202.

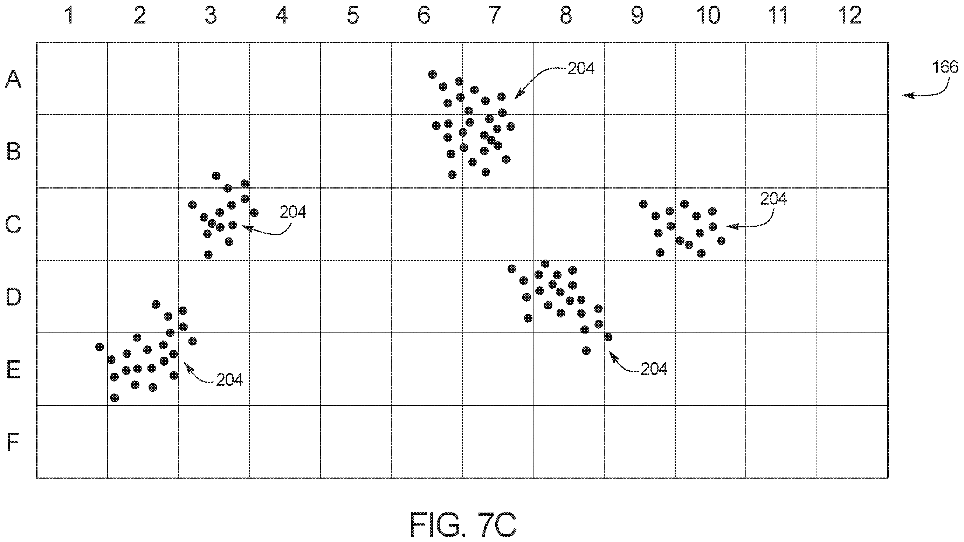

FIG. 7C illustrates target zone 166 during a PCR. As illustrated, the molecules shown in bins B7, C3, C10, D8 and E2 in FIG. 7B have multiplied by diffusion. Clear colonies 204 of nucleic acid molecules 202 can be viewed because the diffusion speed of the molecules is slower than the speed required for the molecules to travel the distance between colonies. Bins A6, A7, B3, B6, B7, C3, C4, C9, C10, D2, D3, D7, D8, E1, E2, E3, E8 and E9 each contain nucleic acid molecules 202 in FIG. 7C.

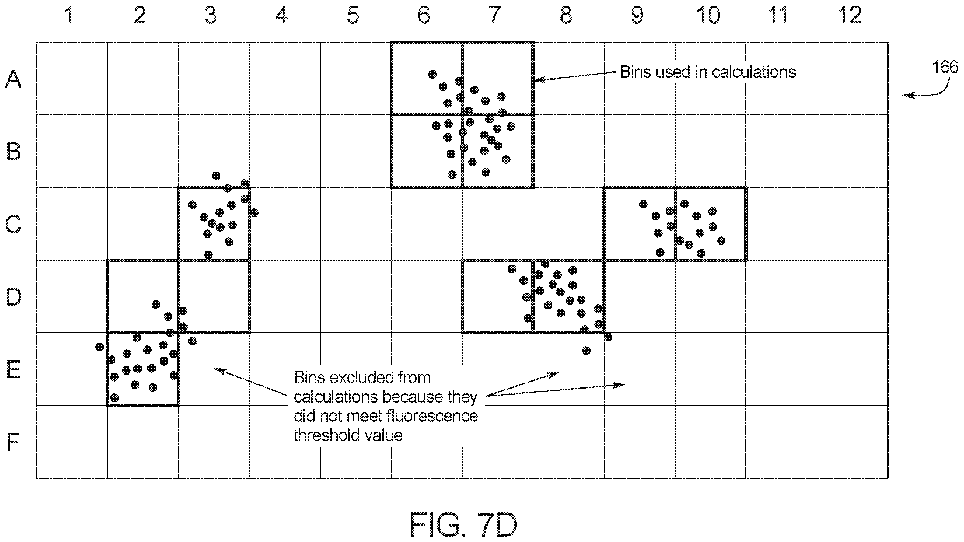

FIG. 7D illustrates bins 200 that have been selected by controller 28 to be used in calculations for amplification and melt curves and for the corresponding analysis. In the illustrated embodiment, the colonies 204 of nucleic acid molecules 202 are visualized by controller 28 based on localized increases in brightness in each bin. If a bin 200 reaches a threshold value for brightness, then that bin's fluorescence value is used in calculations for amplification and melt curves and for the corresponding analysis. If a bin 200 does not reach the threshold value for brightness, then that bin 200 is excluded from the calculations for amplification and melt curves and for the corresponding analysis. In order to get a final value for fluorescence of the entire target zone 166, the arithmetic mean is taken between all of the selected bins 200. In the illustrated embodiment, bins A6, A7, B6, B7, C3, C9, C10, D2, D3, D7, D8 and E2 have been selected for use in calculations for amplification and melt curves and for the corresponding analysis, while bins B3, C4, E1, E3, E8 and E9 have been excluded even though they contain nucleic acid molecules because they do not meet the threshold for brightness.

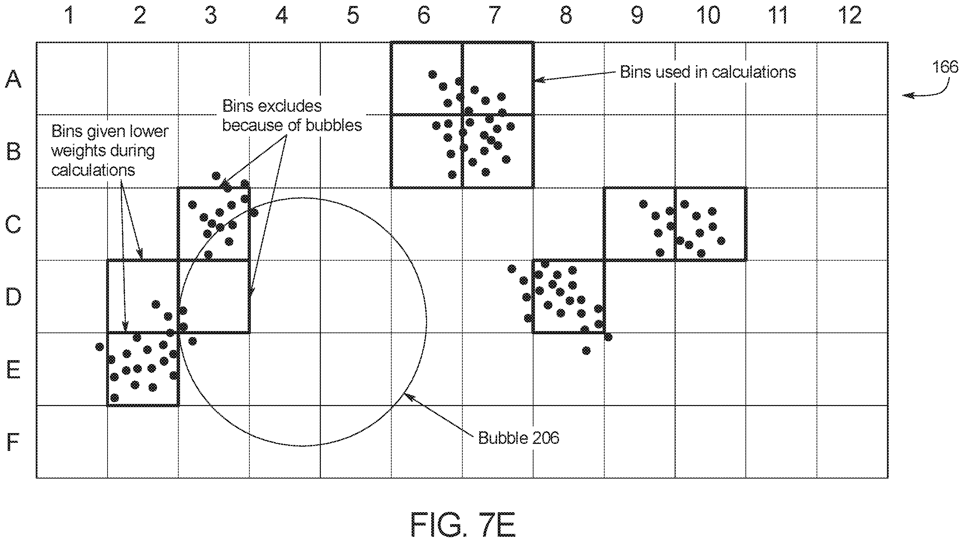

FIG. 7E illustrates how an unwanted object such as a bubble affects the analysis by controller 28. In the illustrated embodiment, a bubble 206 has been detected by controller 28 in bins C3, C4, C5, C6, D3, D4, D5, D6, E3, E4, E5, E6, F3, F4 and F5. Even though bins C3 and D3 met the threshold value for brightness, controller 28 has determined that bins C3 and D3 should be excluded from the amplification and melt curves and corresponding analysis due to the presence of bubble 206. Controller 28 has also determined that bins D2 and E2 should be given a lower weight during calculations due to their proximity to bubble 206.

Based on the above analysis, controller 28 can average the fluorescence values of the selected bins 200. If controller 28 does not detect any bubbles in the images taken by camera imaging device 20, then controller 28 calculates the fluorescence using the following equation:

$$F = \frac{\sum_{i=1}^{n} x_i}{n},$$

wherein n is the number of bins 200 used in the calculation, and $x_i$ is the individual bins in the grid utilized by the algorithm.

If one or more bubble is detected in target zone 166, then controller 28 calculates the fluorescence using the following equation:

$$F = \frac{\sum_{i=1}^{n} a_i x_i}{n},$$

where $a_i$ is the bin's weight, which is determined by the bin's proximity to the bubble. In an aspect of the disclosure, $a_i$ should be greater than zero and less than or equal to one ($0 < a_i \leq 1$).

In an aspect of the disclosure, the $a_i$'s may all have an equal weight of 1 when there are no bubbles present. In an aspect of the disclosure, the $a_i$'s value can decrease proportionally as the bubble gets closer to the bin.

The amplification curve and melt curve may be created by measuring the absolute value of the fluorescence of each pixel in successive images taken either during each cycle (for the amplification curve) or as temperature is slowly increased over time (for the melt curve). In the case of an amplification curve, which shows the incremental change in fluorescence during the amplification stage of a PCR, a PCR crossover threshold value can be determined, and controller 28 can look for when the fluorescence value has increased past a certain threshold value and when the first derivative of fluorescence with respect to cycle number is at a maximum. In the case of a melt curve, the incremental change in fluorescence can give controller 28 the melting temperature of the amplified DNA. Mathematically, controller 28 looks for when the first derivative of the fluorescence with respect to temperature is at a minimum, and the second derivative of the fluorescence with respect to temperature is equal to zero.

The above detection and analysis is useful for a fluorescence based PCR or cytometry analysis, but those of ordinary skill in the art will recognize that controller 28 can be used for other purposes. In an aspect of the disclosure, controller 28 can use camera imaging device 20 to perform a colormetric analysis, which analyzes the concentration of a chemical element or chemical compound in a solution with the aid of a color reagent. Controller 28 can quantify the amount of protein present in the fluid sample by measuring the absorption spectra and comparing it with protein solutions of known concentration. In an aspect of the disclosure, controller 28 can analyze and interpret the results of colormetric protein assays, for example, a Bradford protein assay, a bichinchoninin acid assay (BCA assay), and/or a Ponceau S dye assay.

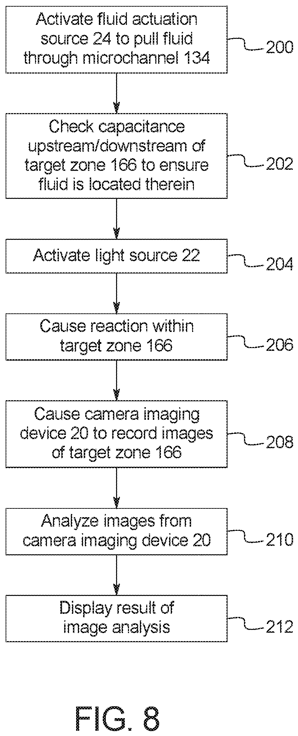

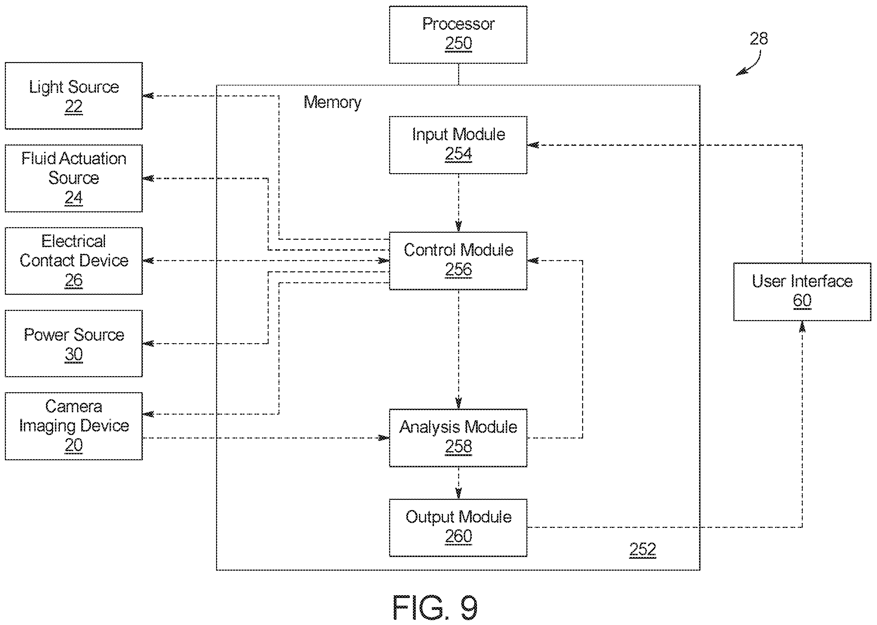

FIG. 8 illustrates an example embodiment of a control method that can be used by controller 28 to perform and analyze a reaction as described herein, and FIG. 9 illustrates an example embodiment of a controller 28 that can perform the method of FIG. 8. As illustrated, controller 28 can include a processor 250 and a memory 252, which can include a non-transitory computer readable medium. Memory 252 can include, for example, an input module 254, a control module 256, an analysis module 258, and an output module 260. Processor 250 can run the modules 252, 254, 256, 258 in accordance with instructions stored on memory 252. The broken lines in FIG. 8 illustrate the electrical connections between the modules 252, 254, 256, 258 of controller 28 and various elements of device 10. It should be understood by those of ordinary skill in the art that the illustrated modules and/or additional modules can be connected to the elements shown and/or additional elements.

The process begins by loading a test card 100 and/or a fluid sample into device 10. The fluid sample can be mixed with a reagent before injection into test card 100 and/or device 10, or can be mixed with a reagent within mixing chamber 126 of test card 100. The reagent may include a PCR inhibitor-resistant polymerase along with a specific mixture of reverse transcriptase (in the case of RNA targets) and surfactants/dispersants to allow for rapid sample dispersion and lysing. The reagent mix may include, for example, oligonucleotide primers, dNTP's DNA polymerase and other chemicals to assist the PCR. It is important to have a correct ratio of fluid sample to final PCR volume, because if the correct ratio is not maintained, the PCR will take too long or fail.

Using user interface 60, a user can then choose a positive/negative test to run on the fluid sample. The user can cycle through a plurality of tests on display 62 using buttons 64 and choose one or more test to run. The plurality of tests can include, for example, a PCR analysis, a cytometry analysis and/or an enzyme-linked immunosorbent assay (ELISA) analysis. In an alternative, a plurality of different types of test cards 100 can be inserted into device 10, with each test card 100 corresponding to one or more specific test to be run on a fluid sample, and controller 28 can determine which test(s) to run by detecting the type of test card 100 inserted into device 10 without further instruction by the user. In another alternative, fluid microchannel 134 can be incorporated into device 10 rather than test card 100, and a user can choose a test to run on the fluid sample after the fluid sample is injected directly into device 10.

Input module 254 is configured to receive the user inputs inputted into user interface 60 and communicate the user inputs to control module 256. Input module 254 can also receive additional information via user interface 60 and/or by the preprogramming of controller 28, for example, (i) real-time PCR crossover threshold value information; (ii) maximum fluorescence information; and (iii) melting curve inflection temperature information. Other control parameters for the PCR reaction can also be given. This includes the PCR denaturing, elongation and annealing temperatures and dwell times.

Once a test card 100 and/or fluid sample has been loaded into device 10, control module 256 of controller 28 begins the control method at step 200 by causing fluid actuation source 26 to pull fluid through microchannel 134. In the illustrated embodiment, fluid actuation source applies a negative pneumatic force to outlet port 130 via pneumatic tube 40 to pull fluid through microchannel 134. In an alternative, fluid actuation source can include one or more other type of pump in fluid communication with microchannel 134.

After fluid actuation source 26 has been activated, but before the reaction begins, control module 256 at step 202 can verify that fluid is located within target zone 166 by monitoring the capacitance of microchannel 134 at one or more locations upstream and/or downstream of target zone 166. If fluid is detected in microchannel 134 upstream and/or downstream of target zone 166, control module 256 can verify that fluid is located within target zone 166, activate light source 22 at step 204, and begin the reaction at step 206. Controller 28 can choose a portion of microchannel 134 to designate as target zone 166, or target zone 166 can be predetermined.

In the illustrated embodiment, control module 256 begins the reaction at step 206 by instructing power source 30 to send a current to electrodes located adjacent to target zone 166 via electrical contact device 26 to cause the fluid within target zone 166 to be heated. As the fluid sample is heated, the nucleic acid molecules multiply by diffusion as explained above.

At the same time that the fluid sample is being heated within target zone 166 so that the nucleic acid molecules multiply by diffusion, control module 256 at step 208 can cause camera imaging device 20 to record a plurality of images of the reaction within target zone 166. The plurality of images can then be sent to analysis module 208 for analysis at step 210. Test card 100 may include a transparent material that allows images to be taken of target zone 166 of fluid microchannel 134 even though a layer of polymer material is located between camera imaging device 20 and fluid microchannel 134.

At step 210, analysis module 258 analyzes the images taken by camera imaging device 20 to determine whether the fluid sample tests positive or negative for a bacteria or virus. The type of analysis performed by analysis module at step 210 will depend on the type of test being run on the fluid sample.

If the assay being run on the fluid sample is a PCR analysis, then analysis module 258 can analyze the images as discussed above by measuring fluorescence based on detected wanted and unwanted objects.

If the assay being run on the fluid sample is a cytometry analysis, then analysis module 258 can also analyze the images as discussed above by measuring fluorescence based on detected wanted and unwanted objects. The cytometry analysis can differ from the PCR analysis, for example, because the fluid in target zone 166 does not need to be heated to multiply molecules by diffusion, so step 206 can be skipped. With a cytometry analysis, analysis module 258 can analyze the fluid sample within target zone 166, for example, by analyzing cell size, cell count, cell morphology (shape and structure), cell cycle phase, DNA content, and the existence or absence of specific proteins on cell surfaces. Controller 28 may use various different fluorophores for flow cytometry.

To detect specific proteins on cell surfaces, specifically designed fluorophores which bind to those proteins may be mixed into the sample to cause the proteins of interest to fluoresce.

If the assay being run on the fluid sample is an ELISA analysis, then again the fluid in target zone 166 does not need to be heated to multiply molecules by diffusion, so step 206 can be skipped. With an ELISA analysis, analysis module 258 can analyze the fluid sample within target zone 166, for example, by measuring the concentration of an analyte in the fluid sample using a colormetric analysis. In an aspect of the disclosure, controller 28 can measure the amount of incident light scattered on the ELISA target zone to determine the concentration of an analyte. The method of measurement is the same as the turbidity measurement.

In an aspect of the disclosure, controller 28 can perform genotyping tests as an extension a PCR.

At step 212, analysis module 258 determines based on the analysis whether the fluid sample has tested positive or negative for a bacteria or virus. The results of the analysis are then displayed on user interface 60 by output module 260. In an aspect of the disclosure, a simple "POSITIVE" or "NEGATIVE" indication can be displayed on user interface 60 to inform whether the fluid sample has tested positive or negative for a bacteria or virus. In another aspect of the disclosure, user interface 60 can display the results for more than one bacteria or virus, or can display specifics such as cell size, cell count, cell morphology (shape and structure), cell cycle phase, DNA content, and the existence or absence of specific proteins on cell surfaces. In an aspect of the disclosure, controller 28 can display viral titer in the case of a PCR reaction, or can display protein concertation and DNA concentration.

In an aspect of the disclosure, the result of the analysis can be saved in a memory module of memory 252, so that the results can be reviewed at a later time. If the result is saved, the result should be encoded to protect the anonymity of the patient. In another aspect of the disclosure, one or more encoded results can be wirelessly transmitted for review at a location remote from diagnostic system 1.

In an aspect of the disclosure, device 10 can include a global positioning system (GPS) sensor, and can record the result of the test along with a GPS sensor reading at the time of the test. Controller 28 can then aggregate a plurality of tests to determine viruses or bacteria that are more prevalent in one area as opposed to another. In this aspect of the disclosure, controller 28 does not need to save any patient information, and only needs the GPS location and the number of positive and negative test results at the location to determine the prevalence of the virus or bacteria at the location. The results can be used by a health organization to treat an area with appropriate medication for a prevalent virus or bacteria.

In another aspect of the disclosure, the user can program the location into device 10 before, during or after running a plurality of tests, and controller 28 can aggregate the plurality of tests to determine viruses or bacteria that are more or less prevalent in the programmed area.

In an aspect of the disclosure, controller 28 can determine whether one or more of yersina pestis, brucella, alphavirus, dengue virus and/or variola virus is present in the fluid sample.

The diagnostic system 1 disclosed herein can detect, for example, lentiviruses (ssRNA) and adenoviruses (dsDNA) in whole blood, as well as other infectious agents. Exemplary infectious agents which can be detected by the system 1 disclosed herein include, but are not limited to, bacterial pathogens, viral pathogens, fungal pathogens, and/or parasitic pathogens.

Exemplary non-limiting bacterial pathogens include *Bacillus* (e.g., *Bacillus anthracis, Bacillus cereus), Bartonella* (e.g., *Bartonella henselae, Bartonella quintana), Bordatella* (e.g., *Bordatella pertussis), Borrelia* (e.g., *Borrelia burgdorferi, Borrelia garinii, Borrelia afzelii, Borrelia recurrentis), Brucella* (e.g., *Brucella abortus, Brucella canis, Brucella melitensis, Brucella suis), Campylobacter* (e.g., *Campylobacter jejuni), Chlamydia* (e.g., *Chlamydia pneumoniae, Chlamydia trachomatis), Chlamydophila* (e.g., *Chlamydophila psittaci), Clostridium* (e.g., *Clostridium botulinum, Clostridium difficile, Clostridium perfringens, Clostridium tetani), Corynebacterium* (e.g., *Corynebacterium diphtheriae), Enterococcus* (e.g., *Enterococcus faecalis, Enterococcus faecium), Escherichia* (e.g, *Escherichia coli), Francisella* (e.g., *Francisella tularensis), Haemophilus* (e.g., *Haemophilus influenzae), Helicobacter* (e.g., *Helicobacter pylori), Legionella* (e.g., *Legionella pneumophila), Leptospira* (e.g., *Leptospira interrogans, Leptospira santarosai, Leptospira weilii, Leptospira noguchii), Listeria* (e.g., *Listeria monocytogenes), Mycobacterium* (e.g., *Mycobacterium leprae, Mycobacterium tuberculosis, Mycobacterium ulcerans), Mycoplasma* (e.g., *Mycoplasma pneumoniae), Neisseria* (e.g., *Neisseria gonorrhoeae, Neisseria meningitidis), Pseudomonas* (e.g., *Pseudomonas aeruginosa), Rickettsia* (e.g., *Rickettsia rickettsii), Salmonella* (e.g., *Salmonella typhi, Salmonella typhimurium), Shigella* (e.g., *Shigella sonnei), Staphylococcus* (e.g., *Staphylococcus aureus, Staphylococcus epidermidis, Staphylococcus saprophyticus), Streptococcus* (e.g., *Streptococcus agalactiae, Streptococcus pneumoniae, Streptococcus pyogenes), Treponema* (e.g., *Treponema pallidum), Ureaplasma* (e.g., *Ureaplasma urealyticum), Vibrio* (e.g., *Vibrio cholerae),* and *Yersinia* (e.g., *Yersinia pestis, Yersinia enterocolitica, Yersinia pseudotuberculosis).*

Exemplary non-limiting viral pathogens include Adenoviridae (e.g., adenovirus), Herpesviridae (e.g., herpes simplex virus type 1 and type 2, varicella-zoster virus, cytomegalovirus, Epstein-Barr virus, human herpesvirus type 8), Papillomaviridae (e.g., human papillomavirus), Polyomaviridae (e.g., BK virus, JC virus), Poxviridae (e.g., smallpox), Hepadnaviridae (e.g., hepatitis B virus), Parvoviridae (e.g., human bocavirus, parvovirus B19), Astroviridae (e.g., human astrovirus), Caliciviridae, (e.g., Norwalk virus), Picornaviridae (e.g., Coxsackievirus, hepatitis A virus, poliovirus, rhinovirus); Coronaviridae (e.g., severe acute respiratory syndrome virus, Middle East respiratory syndrome virus), Flaviviridae (e.g., hepatitis C virus, yellow fever virus, dengue virus, West Nile virus), Togaviridae (e.g., rubella virus), Hepeviridae (e.g., hepatitis E virus), Retroviridae (e.g., lentiviruses, human immunodeficiency virus); Orthomyxoviridae (e.g., influenza virus), Arenaviridae (e.g., Guanarito virus, Junin virus, Lassa virus, Machupo virus, Sabiá virus), Bunyaviridae (e.g., Crimean-Congo hemorrhagic fever virus), Filoviridae (e.g., Ebola virus, Marburg virus), Paramyxoviridae (e.g., measles virus, mumps virus, parainfluenza virus, respiratory syncytial virus, human metapneumonia virus, Hendra virus, Nipah virus), Phabdoviridae (e.g., rabies virus), Reoviridae (e.g., rotavirus, orbivirus, coltivirus, Banna virus), and unassigned viruses (e.g., Hepatitis D virus).

Exemplary non-limiting fungal pathogens include *Candida* (e.g., *Candida albicans), Aspergillus* (e.g., *Aspergillus fumigatus, Aspergillus flavus), Crytopcoccus* (e.g., *Cryptococcus neoformans, Cryptococcus laurentii, Cryptococcus gattii), Histoplasma* (e.g., *Histoplasma capsulatum), Pneumocystis* (e.g., *Pneumocystis jirovecii, Pneumocystis carinii), Stachybotrys* (e.g., *Stachybotrys chartarum).*

Exemplary non-limiting parasitic pathogens include acanthamoeba, anisakis, *Ascaris lumbricoides,* botfly, *Balantidium coli,* bedbugs, *Cestoda* (tapeworm), chiggers, *Cochliomyia hominivorax, Entamoeba histolytica, Fasciola hepatica, Giardia lamblia,* hookworms, *Leishmania, Linguatula serrata,* liver flukes, *Loa loa, Paragonimus* - lung fluke, pinworm, *Plasmodium falciparum, Schistosoma, Strongyloides stercoralis,* mites, tapeworms, *Toxoplasma gondii, Trypanosoma,* whipworms, and *Wuchereria bancrofti.*

Unless otherwise indicated, all numbers expressing quantities of ingredients, properties such as molecular weight, reaction conditions, and so forth used in the specification and claims are to be understood as being modified in all instances by the term "about." Accordingly, unless indicated to the contrary, the numerical parameters set forth in the following specification and attached claims are approximations that may vary depending upon the desired properties sought to be obtained by the present disclosure. At the very least, and not as an attempt to limit the application of the doctrine of equivalents to the scope of the claims, each numerical parameter should at least be construed in light of the number of reported significant digits and by applying ordinary rounding techniques. Notwithstanding that the numerical ranges and parameters setting forth the broad scope of the disclosure are approximations, the numerical values set forth in the specific examples are reported as precisely as possible. Any numerical value, however, inherently contains certain errors necessarily resulting from the standard deviation found in their respective testing measurements.

The terms "a" and "an" and "the" and similar referents used in the context of the disclosure (especially in the context of the following claims) are to be construed to cover both the singular and the plural, unless otherwise indicated herein or clearly contradicted by context. Recitation of ranges of values herein is merely intended to serve as a shorthand method of referring individually to each separate value falling within the range. Unless otherwise indicated herein, each individual value is incorporated into the specification as if it were individually recited herein. All methods described herein can be performed in any suitable order unless otherwise indicated herein or otherwise clearly contradicted by context. The use of any and all examples, or exemplary language (e.g. "such as") provided herein is intended merely to better illuminate the disclosure and does not pose a limitation on the scope of the disclosure otherwise claimed. No language in the specification should be construed as indicating any non-claimed element essential to the practice of the disclosure.

The use of the term "or" in the claims is used to mean "and/or" unless explicitly indicated to refer to alternatives only or the alternatives are mutually exclusive, although the disclosure supports a definition that refers to only alternatives and "and/or."

Groupings of alternative elements or embodiments of the disclosure disclosed herein are not to be construed as limitations. Each group member may be referred to and claimed individually or in any combination with other members of the group or other elements found herein. It is anticipated that one or more members of a group may be included in, or deleted from, a group for reasons of convenience and/or patentability. When any such inclusion or deletion occurs, the specification is herein deemed to contain the group as modified thus fulfilling the written description of all Markush groups used in the appended claims.

Preferred embodiments of the disclosure are described herein, including the best mode known to the inventors for carrying out the disclosure. Of course, variations on those preferred embodiments will become apparent to those of ordinary skill in the art upon reading the foregoing description. The inventor expects those of ordinary skill in the art to employ such variations as appropriate, and the inventors intend for the disclosure to be practiced otherwise than specifically described herein. Moreover, any combination of the above-described elements in all possible variations thereof is encompassed by the disclosure unless otherwise indicated herein or otherwise clearly contradicted by context.

Specific embodiments disclosed herein may be further limited in the claims using consisting of or consisting essentially of language. When used in the claims, whether as filed or added per amendment, the transition term "consisting of" excludes any element, step, or ingredient not specified in the claims. The transition term "consisting essentially of" limits the scope of a claim to the specified materials or steps and those that do not materially affect the basic and novel characteristic(s). Embodiments of the disclosure so claimed are inherently or expressly described and enabled herein.

Further, it is to be understood that the embodiments of the disclosure disclosed herein are illustrative of the principles of the present disclosure. Other modifications that may be employed are within the scope of the disclosure. Thus, by way of example, but not of limitation, alternative configurations of the present disclosure may be utilized in accordance with the teachings herein. Accordingly, the present disclosure is not limited to that precisely as shown and described.

**Claims**

1. A device (10) for analysing a polymerase chain reaction in a fluid sample, the device comprising:

   a current source (30) configured to cause the polymerase chain reaction by heating the fluid sample within a target zone (166); a camera imaging device (20) configured to record a plurality of images of the fluid sample in the target zone while the current source causes the polymerase chain reaction; and a controller (28) configured to, for at least one of the plurality of images, (i) virtually divide the target zone in the respective image into a plurality of bins (200) without the bins being separated by physical barriers, (ii) distinguish wanted objects in the plurality of bins from an unwanted object in the plurality of bins by comparing a size or shape of objects in the respective image to an average size or an average shape of blood cells, and (iii) determine whether the fluid sample tests positive or negative for a bacteria or virus based on the wanted objects by selecting at least two of the plurality of bins that overlap a cluster of wanted objects and calculating a mean fluorescence value of the at least two of the plurality of bins, wherein the wanted objects include cells, and the unwanted object includes an object that does not have at least one of the average size or the average shape of the cells, and wherein the controller (28) is configured to at least one of (a) exclude at least one of the plurality of bins from the mean fluorescence value calculation if the at least one of the plurality of bins overlaps the unwanted object, or (b) assign a weight to at least one of the plurality of bins used in the mean fluorescence value calculation based on the proximity of the at least one of the plurality of bins to the unwanted object.

2. The device (10) of Claim 1, wherein the unwanted object is an air bubble.

3. The device (10) of Claims 1 or 2, wherein the controller (28) is configured to assign weights to at least two of the plurality of bins based on a proximity of the unwanted object, the weights being greater than zero, and use the weights of the at least two of the plurality of bins in a mean fluorescence value calculation.

4. The device (10) of Claims 1 to 3, wherein the plurality of bins (200) are arranged in a grid with a plurality of rows and columns.

5. The device (10) of Claim 1, wherein the controller (28) is configured to additionally select the at least two of the plurality of bins based on a threshold value for brightness.

6. The device (10) of Claim 1, wherein the controller (28) is configured to additionally exclude at least one of the plurality of bins if the at least one of the plurality of bins does not meet a minimum threshold value for brightness.

7. The device (10) of any of Claims 1 to 5, wherein the controller (28) is configured to report an inconclusive test if the controller identifies at least one unwanted object in the plurality of images.

8. The device (10) of any of Claims 1 to 6, which includes a user interface (60), wherein the controller (28) includes a plurality of preprogrammed analyses that can be performed on the fluid sample, and wherein the user interface is configured to allow a user to select at least one analysis from the plurality of preprogrammed analyses.

9. The device (10) of any of Claims 1 to 7, wherein the plurality of images includes at least one of: (i) a plurality of still images of the polymerase chain reaction recorded by the camera imaging device (20) over a period of time; or (ii) a video image of the polymerase chain reaction recorded by the camera imaging device over the period of time.

10. The device (10) of any of Claims 1 to 8, further comprising a light source (22) that provides a fluorescent excitation light on the fluid sample.

11. The device (10) of any of Claims 1 to 8, wherein the device is configured to receive a test card (100) including the fluid sample, and wherein the target zone is located within the test card.

12. A method of using the device of any of Claims 1 to 11, which includes:

heating the fluid sample in the target zone (166) to cause the polymerase chain reaction; recording the plurality of images of the fluid sample in the target zone during the polymerase chain reaction while a light source (22) provides a fluorescent excitation light on the fluid sample; dividing the target zone into a plurality of bins in at least one of the plurality of images; calculating a fluorescence value using at least two of the plurality of bins for the respective image; and determining whether the fluid sample tests positive or negative for a bacteria or virus based on the calculated fluorescence value by selecting at least two of the plurality of bins that overlap a cluster of wanted objects and calculating a mean fluorescence value of the at least two of the plurality of bins, wherein the wanted objects include cells, and the unwanted object includes an object that does not have at least one of the average size or the average shape of blood cells, and wherein calculating the fluorescence value includes at least one of (i) weighting the at least two of the plurality of bins based on proximity to an unwanted object, or (ii) excluding at least one of the plurality of bins from the fluorescence value calculation if the at least one of the plurality of bins overlaps an unwanted object.

**Patentansprüche**

1. Vorrichtung (10) zum Analysieren einer Polymerasekettenreaktion in einer Fluidprobe, wobei die Vorrichtung umfasst:

eine Stromquelle (30), dazu konfiguriert, die Polymerasekettenreaktion durch Erwärmen der Fluidprobe innerhalb einer Zielzone (166) auszulösen; eine Kameraabbildungsvorrichtung (20), dazu konfiguriert, mehrere Bilder der Fluidprobe in der Zielzone aufzuzeichnen, während die Stromquelle die Polymerasekettenreaktion auslöst; und eine Steuerung (28), dazu konfiguriert, für mindestens eines mehreren Bilder (i) die Zielzone in dem jeweiligen Bild virtuell in mehrere Bins (200) zu unterteilen, ohne dass die Bins durch physische Barrieren getrennt sind, (ii) erwünschte Objekte in den mehreren Bins von einem unerwünschten Objekt der mehreren Bins zu unterscheiden, indem eine Größe oder eine Form von Objekten in dem jeweiligen Bild mit einer durchschnittlichen Größe oder einer durchschnittlichen Form von Blutkörperchen verglichen wird, und (iii) zu Bestimmen, ob ein Test der Fluidprobe auf Bakterien oder Viren positiv oder negativ ausfällt, basierend auf den erwünschten Objekten, durch Auswählen von mindestens zwei der mehreren Bins, die einen Cluster von erwünschten Objekten überlappen, und durch Berechnen eines mittleren Fluoreszenzwerts der mindestens zwei der mehreren Bins,

wobei die erwünschten Objekte Zellen einschließen und das unerwünschte Objekt ein Objekt einschließt, das nicht mindestens entweder die durchschnittliche Größe oder die durchschnittliche Form der Zellen aufweist, und wobei die Steuerung (28) konfiguriert ist zum (a) Ausschließen mindestens eines der mehreren Bins von der mittleren Fluoreszenzwertberechnung, wenn der mindestens eine der mehreren Bins das unerwünschte Objekt überlappt, und/oder zum (b) Zuordnen einer Gewichtung zu mindestens einem der mehreren Bins, die bei der Berechnung des mittleren Fluoreszenzwerts verwendet werden, basierend auf der Nähe des mindestens einen der mehreren Bins zu dem unerwünschten Objekt.

2. Vorrichtung (10) nach Anspruch 1, wobei das unerwünschte Objekt eine Luftblase ist.

3. Vorrichtung (10) nach Anspruch 1 oder 2, wobei die Steuerung (28) dazu konfiguriert ist, mindestens zwei der mehreren Bins basierend auf einer Nähe des unerwünschten Objekts Gewichtungen zuzuordnen, wobei die Gewichtungen größer als null sind, und diese Gewichtungen der mindestens zwei der mehreren Bins in einer mittleren Fluoreszenzwertberechnung zu verwenden.

4. Vorrichtung (10) nach Anspruch 1 bis 3, wobei die mehreren Bins (200) in einem Gitter mit mehreren Zeilen und Spalten angeordnet sind.

5. Vorrichtung (10) nach Anspruch 1, wobei die Steuerung (28) dazu konfiguriert ist, zusätzlich die mindestens zwei der mehreren Bins basierend auf einem Helligkeitsschwellenwert auszuwählen.

6. Vorrichtung (10) nach Anspruch 1, wobei die Steuerung (28) dazu konfiguriert ist, mindestens einen der mehreren Bins zusätzlich auszuschließen, wenn der mindestens eine der mehreren Bins einen Mindestschwellenwert für die Helligkeit nicht erfüllt.

7. Vorrichtung (10) nach einem der Ansprüche 1 bis 5, wobei die Steuerung (28) dazu konfiguriert ist, einen nicht schlüssigen Test zu melden, wenn die Steuerung mindestens ein unerwünschtes Objekt in den mehreren Bildern identifiziert.

8. Vorrichtung (10) nach einem der Ansprüche 1 bis 6, die eine Benutzerschnittstelle (60) enthält, wobei die Steuerung (28) mehrere vorprogrammierte Analysen enthält, die an der Fluidprobe durchgeführt werden können, und wobei die Benutzerschnittstelle dazu konfiguriert ist, es einem Benutzer zu ermöglichen, mindestens eine Analyse aus den mehreren vorprogrammierten Analysen auszuwählen.

9. Vorrichtung (10) nach einem der Ansprüche 1 bis 7, wobei die mehreren Bilder Folgendes enthalten: (i) mehrere Standbilder der Polymerasekettenreaktion, die von der Kameraabbildungsvorrichtung (20) über einen Zeitraum aufgezeichnet worden sind; und/oder (ii) ein Videobild der Polymerasekettenreaktion, das von der Kameraabbildungsvorrichtung über den Zeitraum aufgezeichnet worden ist.

10. Vorrichtung (10) nach einem der Ansprüche 1 bis 8, ferner umfassend eine Lichtquelle (22), die ein fluoreszierendes Anregungslicht auf die Fluidprobe bereitstellt.

11. Vorrichtung (10) nach einem der Ansprüche 1 bis 8, wobei die Vorrichtung dazu konfiguriert ist, eine Testkarte (100) mit der Fluidprobe aufzunehmen, und wobei sich die Zielzone innerhalb der Testkarte befindet.

12. Verfahren zum Verwenden der Vorrichtung nach einem der Ansprüche 1 bis 11, umfassend:

Erwärmen der Fluidprobe in der Zielzone (166), um die Polymerasekettenreaktion auszulösen; Aufzeichnen der mehreren Bilder der Fluidprobe in der Zielzone während der Polymerasekettenreaktion, während eine Lichtquelle (22) ein fluoreszierendes Anregungslicht auf die Fluidprobe bereitstellt; Unterteilen der Zielzone in mehrere Bins in mindestens einem der mehreren Bilder; Berechnen eines Fluoreszenzwerts unter Verwendung von mindestens zwei der mehreren Bins für das jeweilige Bild; und Bestimmen, ob ein Test der Fluidprobe auf Bakterien oder Viren positiv oder negativ ausfällt, basierend auf dem berechneten Fluoreszenzwert, durch Auswählen von mindestens zwei der mehreren Bins, die einen Cluster von erwünschten Objekten überlappen, und durch Berechnen eines mittleren Fluoreszenzwerts der mindestens zwei der mehreren Bins, wobei die erwünschten Objekte Zellen einschließen und das unerwünschte Objekt ein Objekt einschließt, das nicht mindestens entweder die durchschnittliche Größe oder die durchschnittliche Form von Blutzellen aufweist, und wobei das Berechnen des Fluoreszenzwerts Folgendes umfasst: (i) Gewichten der mindestens zwei der mehreren Bins basierend auf der Nähe zu einem unerwünschten Objekt, und/oder (ii) Ausschließen mindestens eines der mehreren Bins von der Fluoreszenzwertberechnung, wenn der mindestens eine der mehreren Bins ein unerwünschtes Objekt überlappt.

**Revendications**

1. Dispositif (10) destiné à analyser une amplification en chaîne par polymérase dans un échantillon de fluide, le dispositif comprenant :

   une source de courant (30) configurée pour provoquer l'amplification en chaîne par polymérase en chauffant l'échantillon de fluide à l'intérieur d'une zone cible (166) ;
   un dispositif d'imagerie à caméra (20) configuré pour enregistrer une pluralité d'images de l'échantillon de fluide dans la zone cible pendant que la source de courant provoque l'amplification en chaîne par polymérase ; et
   un contrôleur (28) configuré pour, pour au moins une de la pluralité d'images, (i) diviser virtuellement la zone cible dans l'image respective en une pluralité de cases (200) sans que les cases soient séparées par des barrières physiques, (ii) distinguer des objets souhaités dans la pluralité de cases d'un objet indésirable dans la pluralité de cases en comparant une taille ou forme d'objets dans l'image respective à une taille moyenne ou une forme moyenne de cellules sanguines, et (iii) déterminer si l'échantillon de fluide est testé positif ou négatif pour une bactérie ou un virus en fonction des objets souhaités en sélectionnant au moins deux de la pluralité de cases qui chevauchent un groupe d'objets souhaités et en calculant une valeur de fluorescence moyenne des au moins deux de la pluralité de cases,
   dans lequel les objets souhaités incluent des cellules, et l'objet indésirable inclut un objet qui n'a pas la taille moyenne et/ou la forme moyenne des cellules, et
   dans lequel le contrôleur (28) est configuré pour (a) exclure au moins une de la pluralité de cases du calcul de valeur de fluorescence moyenne si l'au moins une de la pluralité de cases chevauche l'objet indésirable, et/ou (b) attribuer un poids à au moins une de la pluralité de cases utilisées dans le calcul de valeur de fluorescence moyenne en fonction de la proximité de l'au moins une de la pluralité de cases avec l'objet indésirable.

2. Dispositif (10) de la revendication 1, dans lequel l'objet indésirable est une bulle d'air.

3. Dispositif (10) des revendications 1 et 2, dans lequel le contrôleur (28) est configuré pour attribuer des poids à au moins deux de la pluralité de cases en fonction de la proximité de l'objet indésirable, les poids étant supérieurs à zéro, et utiliser les poids des au moins deux de la pluralité de cases dans un calcul de valeur de fluorescence moyenne.

4. Dispositif (10) des revendications 1 à 3, dans lequel la pluralité de cases (200) sont disposées dans une grille avec une pluralité de rangées et de colonnes.

5. Dispositif (10) de la revendication 1, dans lequel le contrôleur (28) est configuré pour sélectionner également les au moins deux de la pluralité de cases en fonction d'une valeur seuil pour la luminosité.

6. Dispositif (10) de la revendication 1, dans lequel le contrôleur (28) est configuré pour exclure également au moins une de la pluralité de cases si l'au moins une de la pluralité de cases ne respecte pas une valeur seuil minimale pour la luminosité.

7. Dispositif (10) de l'une quelconque des revendications 1 à 5, dans lequel le contrôleur (28) est configuré pour signaler un test non concluant si le contrôleur identifie au moins un objet indésirable dans la pluralité d'images.

8. Dispositif (10) de l'une quelconque des revendications 1 à 6, qui comporte une interface utilisateur (60), le contrôleur (28) comportant une pluralité d'analyses préprogrammées qui peuvent être effectuées sur l'échantillon de fluide, et l'interface utilisateur étant configurée pour permettre à un utilisateur de sélectionner au moins une analyse parmi la pluralité d'analyses préprogrammées.

9. Dispositif (10) de l'une quelconque des revendications 1 à 7, dans lequel la pluralité d'images inclut : (i) une pluralité d'images fixes de l'amplification en chaîne par polymérase enregistrées par le dispositif d'imagerie à caméra (20) sur un laps de temps ; et/ou (ii) une image vidéo de l'amplification en chaîne par polymérase enregistrée par le dispositif d'imagerie à caméra sur le laps de temps.

10. Dispositif (10) de l'une quelconque des revendications 1 à 8, comprenant en outre une source de lumière (22) qui projette une lumière d'excitation de fluorescence sur l'échantillon de fluide.

11. Dispositif (10) de l'une quelconque des revendications 1 à 8, le dispositif étant configuré pour recevoir une carte de test (100) comportant l'échantillon de fluide, et la zone cible étant située à l'intérieur de la carte de test.

12. Procédé d'utilisation du dispositif de l'une quelconque des revendications 1 à 11, qui comporte :

le chauffage de l'échantillon de fluide dans la zone cible (166) pour provoquer l'amplification en chaîne par polymérase ;

l'enregistrement de la pluralité d'images de l'échantillon de fluide dans la zone cible pendant l'amplification en chaîne par polymérase pendant qu'une source de lumière (22) projette une lumière d'excitation de fluorescence sur l'échantillon de fluide ;

la division de la zone cible en une pluralité de cases dans au moins une de la pluralité d'images ;

le calcul d'une valeur de fluorescence au moyen d'au moins deux de la pluralité de cases pour l'image respective ; et

la détermination que l'échantillon de fluide est testé positif ou négatif pour une bactérie ou un virus en fonction de la valeur de fluorescence calculée par sélection d'au moins deux de la pluralité de cases qui chevauchent un groupe d'objets souhaités et calcul d'une valeur de fluorescence moyenne des au moins deux de la pluralité de cases,

dans lequel les objets souhaités incluent des cellules, et l'objet indésirable inclut un objet qui n'a pas la taille moyenne et/ou la forme moyenne de cellules sanguines, et

dans lequel le calcul de la valeur de fluorescence comporte (i) la pondération des au moins deux de la pluralité de cases en fonction de la proximité avec un objet indésirable, et/ou (ii) l'exclusion d'au moins une de la pluralité de cases du calcul de valeur de fluorescence si l'au moins une de la pluralité de cases chevauche un objet indésirable.

FIG. 1

EP 3 310 899 B1

FIG. 2

FIG. 3

FIG. 4

FIG. 5

FIG. 6A

FIG. 6B

FIG. 6C

FIG. 7A

EP 3 310 899 B1

166

202

202

202

202

202

1 2 3 4 5 6 7 8 9 10 11 12

A B C D E F

FIG. 7B

FIG. 7C

EP 3 310 899 B1

FIG. 7D

FIG. 7E

```
┌─────────────────────────┐
│   Activate fluid actuation │
│   source 24 to pull fluid  │──── 200
│  through microchannel 134  │
└─────────────────────────┘
             │
             ▼
┌─────────────────────────┐
│     Check capacitance      │
│ upstream/downstream of     │──── 202
│ target zone 166 to ensure  │
│   fluid is located therein │
└─────────────────────────┘
             │
             ▼
┌─────────────────────────┐
│   Activate light source 22 │──── 204
└─────────────────────────┘
             │
             ▼
┌─────────────────────────┐
│   Cause reaction within    │──── 206
│      target zone 166       │
└─────────────────────────┘
             │
             ▼
┌─────────────────────────┐
│   Cause camera imaging     │
│ device 20 to record images │──── 208
│   of target zone 166       │
└─────────────────────────┘
             │
             ▼
┌─────────────────────────┐
│     Analyze images from    │──── 210
│  camera imaging device 20  │
└─────────────────────────┘
             │
             ▼
┌─────────────────────────┐
│      Display result of     │──── 212
│      image analysis        │
└─────────────────────────┘
```

# FIG. 8

FIG. 9

28

Processor 250

Memory

Input Module 254

Control Module 256

Analysis Module 258

Output Module 260

252

User Interface 60

Light Source 22

Fluid Actuation Source 24

Electrical Contact Device 26

Power Source 30

Camera Imaging Device 20

28

**EP 3 310 899 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2007035172 A
- WO 2015032835 A
- WO 2014055963 A
- US 185640
- US 20160369322 A1
- US 185661
- US 20160369323 A1